# EUROPEAN PATENT SPECIFICATION

(11) **EP 2 851 007 B1**
(45) Date of publication and mention of the grant of the patent: **28.03.2018**
(21) Application number: 13790859.6
(22) Date of filing: 13.05.2013
(51) Int. Cl.: A61B 17/34, A61B 34/30

(54) **TROCAR**
TROKAR
TROCART

(30) Priority: 18.05.2012 JP 2012114445
(43) Date of publication of application: 25.03.2015
(73) Proprietor: Olympus Corporation, Hachioji-shi, Tokyo 192-8507 (JP)
(72) Inventor: KISHI, Kosuke, Hachioji-shi, Tokyo 192-8507 (JP)
(74) Representative: Schicker, Silvia
(86) International application number: PCT/JP2013/063257
(87) International publication number: WO 2013/172289

(56) References cited:
- EP-A1- 2 119 404
- EP-A1- 2 226 026
- WO-A1-2011/062768
- JP-A- 2010 207 578
- JP-A- 2010 207 579
- JP-A- 2011 206 312
- US-A1- 2011 015 491
- US-A1- 2011 015 491

## Description

### Technical Field

The present invention relates to a trocar.

Priority is claimed on Japanese Patent Application No. 2012-114445, filed May 18, 2012.

### Background Art

In the related art, there is known a procedure of forming a small incision for allowing a treatment instrument having an elongated insertion part or an elongated surgical instrument, such as a laparoscope apparatus, to be inserted therethrough, in a patient's abdominal wall, to thereby suppress invasiveness to a patient, instead of making a surgical incision in the abdomen, when an operation is performed.

For example, in the case of minimally invasive surgery when performing treatment within an abdominal cavity, it is common to attach a plurality of trocars to an abdominal wall and to introduce surgical instruments into the abdominal cavity through the trocars. Additionally, as a procedure of further reducing invasiveness to a patient, there is known a procedure of attaching a trocar, to which a plurality of ports are provided, to a patient's navel or the like and inserting surgical instruments into respective ports of the trocar. By using such a trocar, the plurality of surgical instruments can be introduced into the inside of the body through one trocar.

As an example of the trocar used when the plurality of surgical instruments are introduced into the inside of the body through one trocar, Japanese unexamined patent application, first publication no. JP 2011-98138 discloses an insertion instrument (trocar) including a plate member having a plurality of ports formed therein, and a cylindrical member to which the plate member is rotatably attached.
US 2011/015491 A1 discloses a surgical access device for use in laparoscopic surgical procedures including a housing portion and at least one moveable device port. The housing portion has an axial bore extending therethrough, defining a central axis of the surgical access device. The at least one moveable device port is moveable about an annular path corresponding to an annular seal member. Each moveable device port defines a lumen extending therethrough, and each lumen is substantially parallel to the central axis of the surgical access device.

### Summary of the Invention

### Problem to be Solved by the Invention

However, in the technique disclosed in JP 2011-98138, the relative positional relationship between the plurality of ports does not change because the plate member having the plurality of ports formed therein only rotates relative to the cylindrical member. Accordingly, when the surgical instruments are inserted into the plurality of ports, and used respectively, the respective surgical instruments may not be able to be arranged at optimal positions for the treatment of a treatment target part. In US 2011/015491, the surgical access device does not include a locking portion so that the position of the movable port does not deviate easily.

The present invention has been made in view of the above-described circumstances, and an object thereof is to provide a trocar that can easily arrange a plurality of surgical instruments at optimal positions in order to treat a treatment target part.

### Means for solving the Problem

In order to solve the above object, the present invention suggests a trocar according to claim 1. A further aspect of the present invention is provided in the dependent claim.

According to an example useful for understanding the invention, a first surgical instrument attached to a robot may be inserted through a first through-hole formed in a plate member, a second surgical instrument attached to the robot may be inserted through a second through-hole provided in a movable port, and the movable port may be moved relative to the plate member by the robot.

### Advantageous Effects of the Invention

According to the above trocar, a plurality of surgical instruments can be easily arranged at optimal positions for the treatment of a treatment target part.

### Brief Description of the Drawings

FIG. 1 is a perspective view illustrating a trocar of a first embodiment of the present invention.
FIG. 2 is a front view illustrating the trocar.
FIG. 3 is a cross-sectional view taken along line A-A of FIG. 2.
FIG. 4 is a schematic view illustrating one process when the trocar is used.
FIG. 5 is a schematic view illustrating one process when the trocar is used.
FIG. 6 is a schematic view illustrating one process when the trocar is used.
FIG. 7 is a front view of the trocar illustrating the configuration of a modified example of the embodiment.
FIG. 8 is a cross-sectional view taken along line B-B of FIG. 7.
FIG. 9 is a view for describing the operation of the trocar.
FIG. 10 is a cross-sectional view taken along line C-C of FIG. 9.
FIG. 11 is a front view showing a trocar of an example useful for understanding the invention.
FIG. 12 is a schematic view illustrating one process when the trocar is used.
FIG. 13 is a schematic view illustrating one process when the trocar is used.
FIG. 14 is a front view of the trocar illustrating the configuration of a modified example of the example useful for understanding the invention.

### Description of Embodiments

### (First Embodiment)

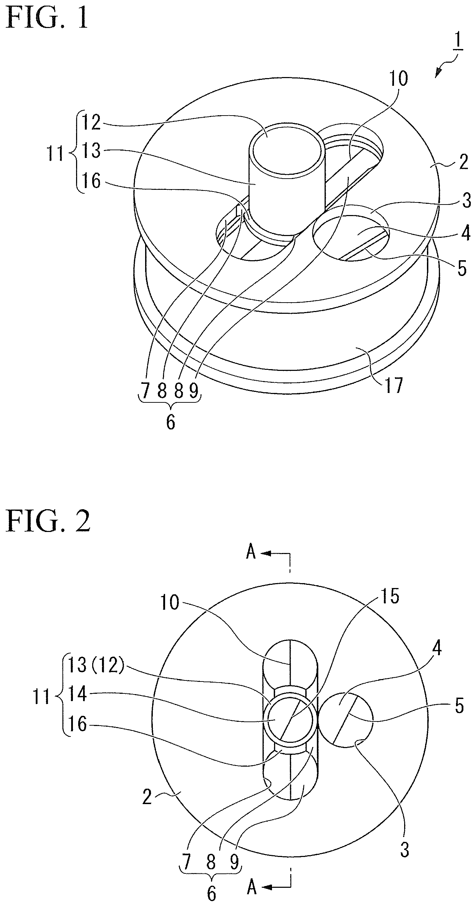

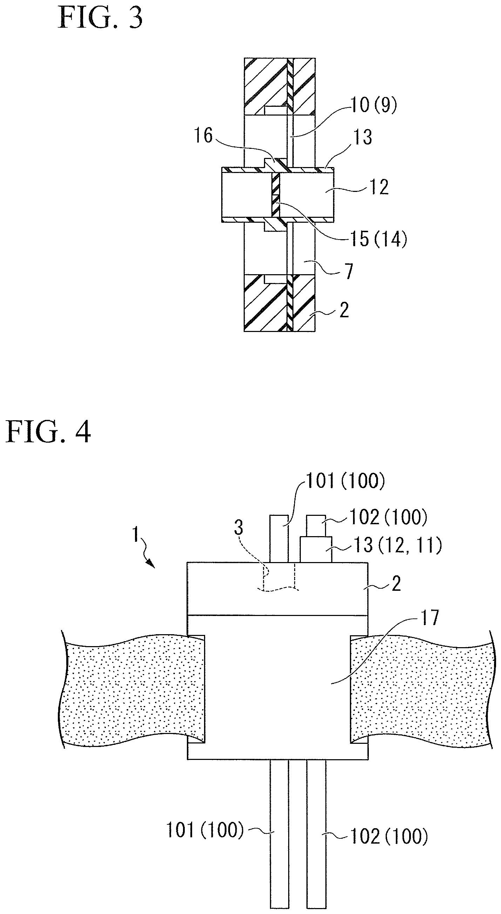

A trocar of a first embodiment of the present invention will be described. FIG. 1 is a perspective view illustrating a trocar of the present embodiment. FIG. 2 is a front view illustrating the trocar. FIG. 3 is a cross-sectional view taken along line A-A of FIG. 2.

As shown in FIG. 1, the trocar 1 of the present embodiment includes a plate member 2, a movable port 11, and a cylinder part 17.

As shown in FIGS. 1 and 2, the plate member 2 is a substantially disk-shaped member in which a through-hole (a first surgical instrument port 3 or a first through-hole) for allowing a surgical instrument to be inserted therethrough is formed. The first surgical instrument port 3 is a hole in which an opening having a circular cross-section is formed, and one port is formed in the plate member 2. The configuration of a surgical instrument to be inserted through the first surgical instrument port 3 is not particularly limited. For example, a treatment instrument, having an elongated insertion part, a laparoscope, or the like can be inserted through the first surgical instrument port 3. Additionally, the first surgical instrument port 3 is provided with an airtight valve 4 in which a slit 5 is formed.

Additionally, the plate member 2 has a guide hole part 6 through which the movable port 11 is inserted, at a position spaced apart from the first surgical instrument port 3.

The guide hole part 6 has an elongated hole portion 7 that allows the movable port 11 to advance and retreat in a predetermined direction, locking portions 8 that lock the movable port 11 to a part of the guide hole part 6, and an airtight valve 9 that airtightly seals the elongated hole portion 7.

As shown in FIG. 2, the elongated hole portion 7 is a hole that is elongated in one predetermined direction (this direction is hereinafter referred to as a "longitudinal direction of the elongated hole portion 7") as is viewed from a thickness direction of the plate member 2 (viewed from a top surface). The longitudinal direction of the elongated hole portion 7 is a direction orthogonal to the central axis of the plate member 2. In addition, the longitudinal direction of the elongated hole portion 7 is appropriately selected and set in consideration of the movement direction of the movable port 11 with respect to the first surgical instrument port 3. Additionally, the elongated hole portion 7 may be a curved line in its longitudinal direction. For example, the elongated hole portion 7 may be formed in a curved shape, such as a circular-arc elongated hole centered on the central axis of the through-hole, an S-shape, a J-shape, or a U-shape.

Each locking portion 8 has a bulged shape formed at an intermediate portion of the elongated hole portion 7 in the longitudinal direction, and is formed so as to slide on an outer surface of the movable port 11 when the movable port 11 moves with respect to the plate member 2. The elongated hole portion 7 and the movable port 11 have little sliding resistance at both ends of the elongated hole portion 7 in the longitudinal direction, as compared to the locking portion 8. Accordingly, when the movable port 11 is moved with respect to the plate member 2 from one end 7a of the elongated hole portion 7 to the other end 7b thereof in the longitudinal direction or to its opposite direction, it is necessary to move the movable port 11 with the capability of exceeding sliding resistance against the locking portion 8. By providing the locking portion 8, a possibility that the movable port 11 may be moved to the intermediate portion of the elongated hole portion 7 in the longitudinal direction by the weight of movable port 11 itself is suppressed to be low. As a result, the movable port 11 is locked to the one end 7a or the other end 7b of the elongated hole portion 7.

The airtight valve 9 provided in the elongated hole portion 7 is a membrane-like member having flexibility. The airtight valve 9 is formed with a slit 10 extending in the longitudinal direction of the elongated hole portion 7. The movable port 11 is inserted through the slit 10 of the airtight valve 9 so as to expand the slit. Accordingly, the region of the elongated hole portion 7 outside a portion into which the movable port 11 is inserted is brought into an airtight state by the airtight valve 9.

As shown in FIGS. 2 and 3, the movable port 11 is provided separately from the plate member 2 and moves relative to the plate member 2. The movable port 11 includes a main body 13 in which a through-hole (a second surgical instrument port 12 or a second through-hole) for allowing a surgical instrument to be inserted therethrough is provided, and a flap portion 16 that is formed to overhang radially outward of the main body 13.

The main body 13 is formed in the shape of a cylinder, and a hole in which an opening having a circular cross-section is formed serves as the second surgical instrument port 12. A surgical instrument capable of being inserted through the second surgical instrument port 12 is not particularly limited, which is similar to the first surgical instrument port 3. Additionally, the second surgical instrument port 12 is provided with an airtight valve 14 made of a flexible membrane-like member in which a slit 15 is formed.

The flap portion 16 is an annular member with a size such that the flap portion is engaged with the elongated hole portion 7 formed in the plate member 2, and is integrally molded with the main body 13 in the present embodiment. The flap portion 16 functions as a retainer that prevents the main body 13 from slipping out of the elongated hole portion 7.

The cylinder part 17 shown in FIG. 1 is a cylindrical member. The cylinder part 17 is provided in the plate member 2 so as to communicate with the inside of the first surgical instrument port 3 formed in the plate member 2 and communicates with the inside of the second surgical instrument port 12 formed in the movable port 11. The cylinder part 17 is attached to an incised wound by being inserted into the incised wound serving as an attachment target for the trocar 1 when the trocar 1 is used.

Next, the usage and operation of the trocar 1 will be described. FIGS. 4 to 6 are schematic views illustrating one process when the trocar 1 is used.

In the present process, there is shown an example in which the trocar 1 according to the present invention is used in an operation under endoscopic view in which a surgeon operates by operating an elongated surgical instrument, such as a treatment instrument having an elongated insertion part or a laparoscope apparatus.

First, a suitable site is incised in order to guide the surgical instrument to a part to be treated of a patient's body wall, and an incised wound for allowing the cylinder part 17 of the trocar 1 shown in FIG. 1 to pass therethrough is formed in the patient's body wall. In the present example, a plurality of surgical instruments are introduced into the inside of the body through one incised wound by using the trocar 1. Thus, the incised wound may be formed in one place of the body wall, for example, a patient's navel.

As shown in FIG. 4, mutually different surgical instruments 100 may be simultaneously attached to the first surgical instrument port 3 formed in the plate member 2 and the second surgical instrument port 12 formed in the movable port 11, respectively. For example, a treatment instrument 101, such as forceps, is attached to the first surgical instrument port 3 formed in the plate member 2 as a first surgical instrument, and a laparoscope apparatus 102 is attached to the second surgical instrument port 12 formed in the movable port 11 as a second surgical instrument. Although the initial position of the movable port 11 with respect to the plate member 2 may be anywhere, a case where an operation is performed in a state where the movable port 11 is located at the one end 7a of the elongated hole portion 7 will be described below as an example.

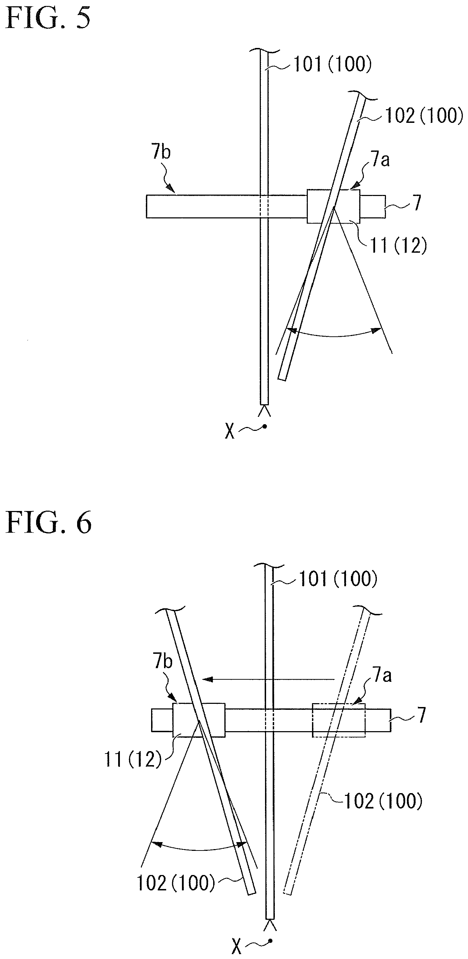

When the treatment instrument 101 introduced into the body through the first surgical instrument port 3 is used to perform a treatment on a treatment target part, as shown in FIG. 5, the treatment proceeds while observing the treatment target part X using the laparoscope apparatus 102 introduced into the body through the second surgical instrument port 12. For example, when the movable port 11 is located at the one end 7a of the elongated hole portion 7, the movable range of the insertion part of the laparoscope apparatus 102 is within a conic region having the one end 7a of the elongated hole portion 7 as a rocking center.

For example, a treatment target part may be observed from a different visual point obtained by changing the visual point of the laparoscope apparatus 102, in a state where treatment is performed on the treatment target part, using the treatment instrument 101 introduced into the body through the first surgical instrument port 3. In this case, the treatment target part may not be able to be appropriately captured within the visual field of the laparoscope apparatus 102, in a state where the movable port 11 is located at the one end 7a of the elongated hole portion 7. Additionally, if it is intended to move the laparoscope apparatus 102 to a desired position in a state where the movable port 11 is located at one end 7a of the elongated hole portion 7, a case where the laparoscope apparatus 102 and the treatment instrument 101 interfere with each other is also considered.

In such a case, as shown in FIG. 6, the movable port 11 is moved from the one end 7a of the elongated hole portion 7 to the other end 7b of the elongated hole portion 7. The movable range of the insertion part of the laparoscope apparatus 102 is within a conic region having the other end 7b of the elongated hole portion 7 as a rocking center. Accordingly, the treatment target part can be observed from the direction of a new visual point using the laparoscope apparatus 102.

As described above, according to the present example, the laparoscope apparatus 102 can be easily arranged at an optimal position for the treatment of a treatment target part. Accordingly, the treatment instrument 101 and the laparoscope apparatus 102 can be moved relative to each other so that the treatment instrument 101 and the laparoscope apparatus 102 establish a suitable positional relationship.

Additionally, since the movable port 11 is inserted through the elongated hole portion 7 and the movable port 11 moves along the elongated hole portion 7, the movable port 11 is brought into a state of being supported by the elongated hole portion 7. For this reason, there is only a little shaking of the surgical instrument 100 attached to the movable port 11. For example, when the laparoscope apparatus 102 is attached to the movable port 11, an image obtained using the laparoscope apparatus 102 is not easily blurred.

Moreover, since the elongated hole portion 7 is provided with the locking portions 8, the movable port 11 is locked to the elongated hole portion 7. For example, in the present example, the movable port 11 is locked to the one end 7a or the other end 7b of the elongated hole portion 7. Thus, when the surgical instrument 100, such as the laparoscope apparatus 102, is moved with the one end 7a or the other end 7b of the elongated hole portion 7 as a rocking center, the position of the movable port 11 does not deviate easily.

For example, in the related art, if the laparoscope apparatus 102 moves unintentionally during treatment, the treatment should be interrupted until the position of the laparoscope apparatus 102 is returned so that a treatment target part falls within a visual field. In contrast, in the trocar 1 of the present inveniton, unintended movement of the laparoscope apparatus 102 does not take place easily. Thus, the treatment can be performed with ease.

### (Modified Example 1)

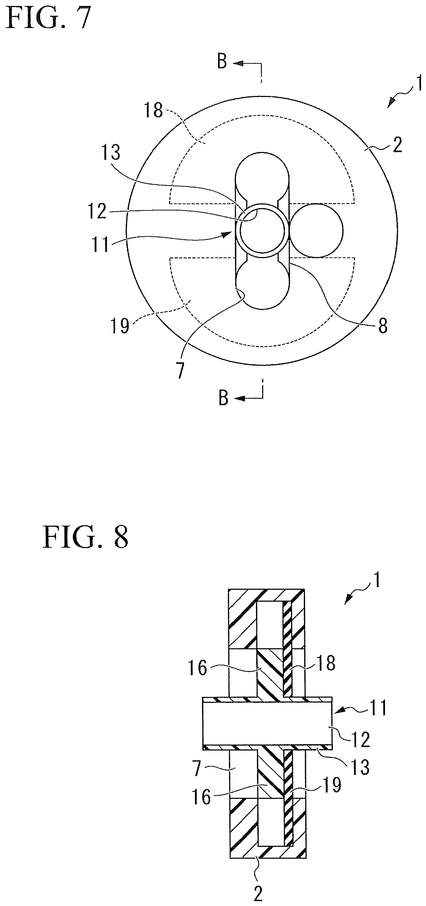

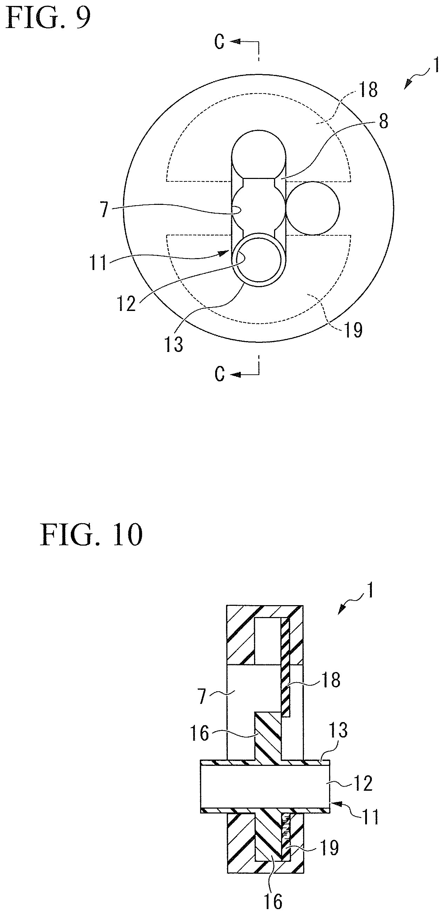

Next, a modified example of the above-described first embodiment will be described. FIG. 7 is a front view of a trocar illustrating the configuration of the present modified example. FIG. 8 is a cross-sectional view taken along line B-B of FIG. 7. FIG. 9 is a view for describing the operation of the trocar. FIG. 10 is a cross-sectional view taken along line C-C of FIG. 9.

As shown in FIGS. 7 and 8, in the present modified example, rubber sheets 18 and 19 that cover a part of the elongated hole portion 7 are provided instead of the airtight valve 9 that seals the elongated hole portion 7. The rubber sheets 18 and 19 are laminated, sheet-like, or film-like members that are elastically deformed when being pressed by the main body 13 of the movable port 11. In the present modified example, the two rubber sheets 18 and 19 formed in the shape of a semicircle are arranged to face the plate member 2, in a state where a gap is formed at a central portion of the elongated hole portion 7 in the longitudinal direction. Additionally, the main body 13 of the movable port 11 is disposed at the gap between the two rubber sheets 18 and 19.

Moreover, in the present modified example, each locking portion 8 is formed such that a central portion is recessed, so that the movable port 11 can be locked to the center of the elongated hole portion 7.

As shown in FIGS. 9 and 10, in the present modified example, the rubber sheet 19 is elastically deformed if the movable port 11 moves to the rubber sheet 19 side. Additionally, an airtight state is maintained by the rubber sheet 18 and the flap portion 16.

Even in such a configuration, the same effects as in the first embodiment are exhibited.

### (Example useful for understanding the invention)

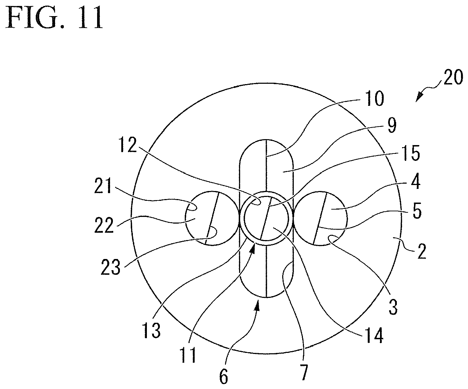

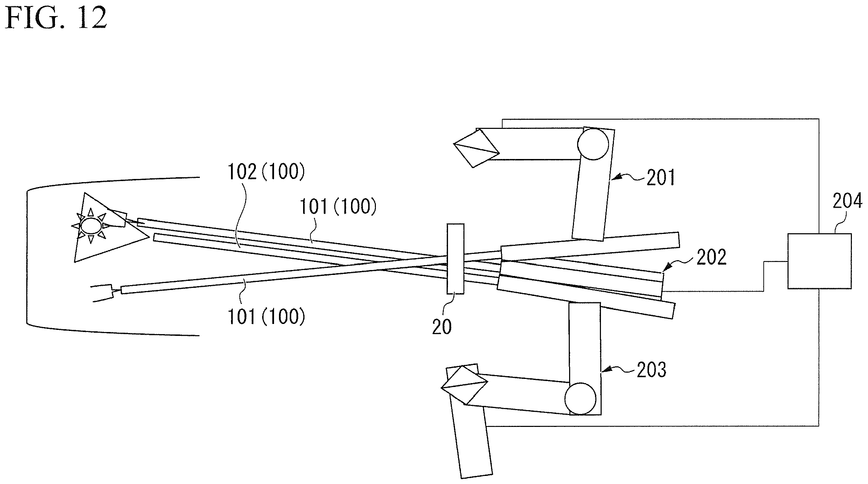

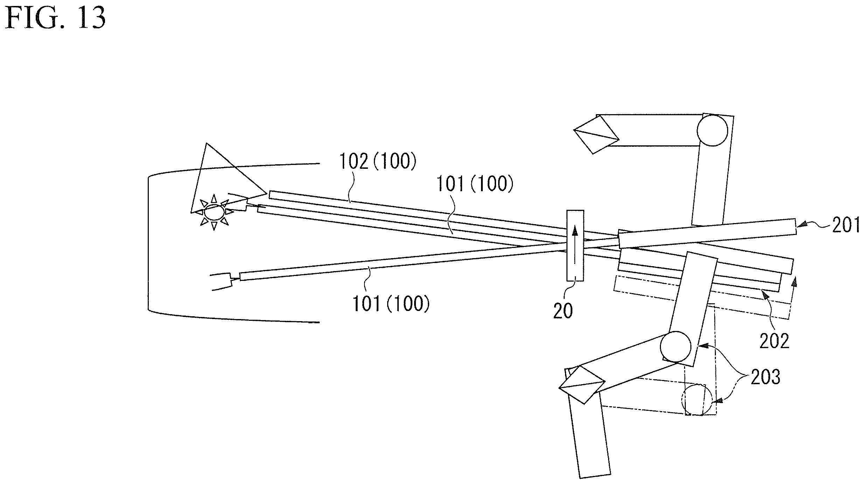

Next, a trocar of an example useful for understanding the invention of the present invention will be described. FIG. 11 is a front view illustrating the trocar of the example useful for understanding the invention. FIGS. 12 and 13 are schematic views illustrating one process when the trocar is used.

The trocar of the example useful for understanding the invention is a trocar that is suitably applied to an operation using a robot.

As shown in FIG. 11, the configuration of the plate member 2 is different from that of the above-described first embodiment in that a trocar 20 of the example useful for understanding the invention does not have the locking portions 8, and a through-hole (a third surgical instrument port 21 or the first through-hole) separate from the first surgical instrument port 3 is further formed in the plate member 2.

The movable port 11 is capable of advancing and retreating in the longitudinal direction of the elongated hole portion 7 provided in the plate member 2.

The third surgical instrument port 21 is arranged opposite to the first surgical instrument port 3 so that the movable port 11 is pinched between the surgical instrument ports. The third surgical instrument port 21 is provided with an airtight valve 22 having a slit 23 similar to the first surgical instrument port 3.

Next, the usage and operation of the trocar 20 will be described.

First, the overview of a robot that performs an operation using the trocar 20 will be described. The configuration of the robot is not particularly limited, and if a robot that operates a plurality of surgical instruments is provided, the trocar 20 of the example useful for understanding the invention can be suitably handled.

As an example of the robot, as shown in FIG. 12, the robot includes two arms 201 and 202 to which the treatment instrument 101 is attached, one arm 203 to which the laparoscope apparatus 102 is attached, and a control device 204 that operates the respective arms 201, 202, and 203.

The two arms 201 and 202 to which the treatment instrument 101 is attached are provided corresponding to the right hand and left hand of a user who operates the robot. Additionally, each of a total of three arms 201, 202, and 203 is a multi-joint arm, and is controlled by the control device 204 so as to move the treatment instrument 101 and the laparoscope apparatus 102 with the first surgical instrument port 3 and the second surgical instrument port 12 as a rocking center. Specifically, a robot including the configuration described in Japanese Unexamined Patent Application, First Publication No. 2000-300579 or the like may be adopted. In addition, the robot that performs an operation using the trocar 20 of the example useful for understanding the invention may be a robot provided with three or more arms.

Also in an operation using the robot, the visual point of the laparoscope apparatus 102 may be changed similarly to the above first embodiment. As shown in FIGS. 12 and 13, in the example useful for understanding the invention, for example, when the laparoscope apparatus 102 is attached to the second surgical instrument port 12 of the movable port 11, the robot moves the laparoscope apparatus 102, and the movable port 11 is moved in the longitudinal direction of the elongated hole portion 7 by the laparoscope apparatus 102. Additionally, in surgical robots that have been known up to now, the laparoscope apparatus 102 can be positioned and can be held for a long time, and the movable port 11 can be positioned and held at arbitrary positions in the elongated hole portion 7. Moreover, the laparoscope apparatus 102 can also be rocked using a position where the movable port 11 is positioned, as a rocking center.

Additionally, in the example useful for understanding the invention, when the robot is being operated in a state where the treatment instrument 101 attached to the first surgical instrument port 3 is made to correspond to the right hand, and the treatment instrument 101 attached to the second surgical instrument port 12 is made to correspond to the left hand, the direction of the visual point of the laparoscope apparatus 102 can be changed with the positional relationship between the right hand and the left hand maintained by moving the laparoscope apparatus 102 attached to the movable port 11 along the guide hole part 6. Accordingly, the visual point can be changed with the operation feeling of the surgical instrument 100 maintained.

### (Modified Example 2)

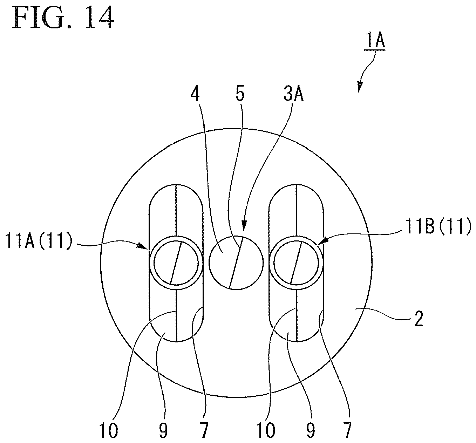

Next, a modified example of the example useful for understanding the invention will be described. FIG. 14 is a front view illustrating a trocar of the example useful for understanding the invention.

As shown in FIG. 14, a trocar 1A of the present modified example is different from the above-described respective example useful for understanding the invention in that the trocar 1A has a plurality of the movable ports 11. The trocar 1A has two movable ports 11 (a first movable port 11A and a second movable port 11B), and a first surgical instrument port 3A that has the same configuration as the above-described first surgical instrument port 3 and is disposed at a central portion of the trocar 1A.

In the trocar 1A shown in FIG. 14, two treatment instruments attached to left and right arms of a robot can be respectively inserted into the first movable port 11A and the second movable port 11B and be used. Accordingly, in a case where the respective treatment instruments are inserted into the inside of the body or the like through the first movable port 11A and the second movable port 11B, even when the treatment instruments attached to the robot or robots interfere with each other outside the body, it is possible to move the first movable port 11A or the second movable port 11B or both of the first movable port 11A and the second movable ports 11B, using a robot's driving force, and to avoid the interference between the treatment instruments or between the robots.

Although the embodiments of the present invention have been described above in detail with reference to the drawings, the specific configuration of the present invention is not limited to the embodiments, and design changes or the like are also included without departing from the scope of the present invention as defined by appended claims. For example, the movable port 11 may be formed with a plurality of surgical instrument insertion through-holes. Additionally, the second surgical instrument port 12 provided in the movable port 11 may be parallel to the first surgical instrument port 3 provided in the plate member 2, or the surgical instrument ports may be arranged at positions where the central axes thereof intersect each other or are twisted from each other.

Additionally, the first surgical instrument port 3 also has the same configuration as the movable port 11. In this case, both of the first surgical instrument port 3 and the second surgical instrument port 12 move relative to the plate member 2.

Additionally, instead of the treatment instrument 101, the laparoscope apparatus 102 can also be inserted into the first surgical instrument port 3. Additionally, instead of the laparoscope apparatus 102, the treatment instrument 101 can also be inserted into the second surgical instrument port 12. Additionally, flexible endoscopes may be inserted into the first surgical instrument port 3 and the second surgical instrument port 12, respectively. That is, if surgical instruments having dimensions such that the surgical instruments can be inserted into the first surgical instrument port 3 and the second surgical instrument port 12 are provided, targets to be inserted into the first surgical instrument port 3 and the second surgical instrument port 12 are not particularly limited.

Additionally, although the above-described embodiments have been described in a way that the elongated insertion part of the laparoscope apparatus 102 is formed in a linear shape in order to simplify the description, the trocar of the present invention exhibits the same effects even when a distal end portion of the insertion part is curved or the laparoscope apparatus is a so-called oblique-viewing endoscope or a side-viewing endoscope.

Additionally, the constituent elements shown in the above-described respective embodiments can be suitably combined.

### Industrial Applicability

According to the above trocar, a plurality of surgical instruments can be easily arranged at optimal positions for the treatment of a treatment target part.

### Reference Signs List

1,20: TROCAR
2: PLATE MEMBER
3: FIRST SURGICAL INSTRUMENT PORT
4: AIRTIGHT VALVE
5: SLIT
6: GUIDE HOLE PART
7: ELONGATED HOLE PORTION
8: LOCKING PORTION
9: AIRTIGHT VALVE
10: SLIT
11: MOVABLE PORT
12: SECOND SURGICAL INSTRUMENT PORT
13: MAIN BODY
14: AIRTIGHT VALVE
15: SLIT
16: FLAP PORTION
17: CYLINDER PART
18,19: RUBBER SHEET
21: THIRD SURGICAL INSTRUMENT PORT
22: AIRTIGHT VALVE
23: SLIT
100: SURGICAL INSTRUMENT
101: TREATMENT INSTRUMENT
102: LAPAROSCOPE APPARATUS

## Claims

1. A trocar (1) comprising:
a plate member (2) which has at least one first through-hole (3) formed therein for allowing a first surgical instrument to be inserted therethrough;
at least one movable port (11) which is provided separately from the plate member (2), is movable relative to the plate member (2), and has a second through-hole (12) formed therein for allowing a second surgical instrument to be inserted therethrough; and
a cylinder part (17) which is provided at the plate member (2) so as to communicate with an inside of the first through-hole (3) and an inside of the second through-hole (12), and which is adapted to be attached to an incised wound,
wherein the plate member (2) has a guide hole part (6) through which the movable port (11) is inserted, wherein
the guide hole part (6) includes an elongated hole portion (7) that allows the movable port (11) to advance and retreat in a predetermined direction, wherein the elongated hole portion (7) extends from a first end (7a) to a second end (7b) of the elongated hole portion (7) in a longitudinal direction perpendicular to a central axis of the plate member (2),
**characterized in that** the guide hole part (6) further includes
a locking portion (8) that is configured to lock the movable port (11) to a part of the guide hole part (6), wherein the locking portion (8) is formed at an intermediate portion of the elongated hole portion (7) between the first and the second end of the elongated hole portion (7) in the longitudinal direction, wherein
a sliding resistance in the longitudinal direction between the elongated hole portion (7) and the movable port (11) at both ends (7a, 7b) of the elongated hole portion (7) is smaller compared to a sliding resistance in the longitudinal direction between the locking portion (8) and the movable port (11).

2. The trocar according to Claim 1,
wherein the guide hole part (6) further includes a valve (9) that airtightly seals the elongated hole portion (7), and
wherein a slit (10), through which the movable port (11) is inserted and which extends in the longitudinal direction of the elongated hole portion (7), is formed in the valve (9).

## Patentansprüche

1. Trokar (1), umfassend:
ein Plattenelement (2), das mindestens ein erstes Durchgangsloch (3) aufweist, das darin gebildet ist, um zu ermöglichen, dass ein erstes chirurgisches Instrument durch dieses hindurch eingeführt werden kann;
mindestens einen bewegbaren Stutzen (11), der getrennt von dem Plattenelement (2) bereitgestellt ist, in Bezug auf das Plattenelement (2) bewegbar ist, und in dem ein zweites Durchgangsloch (12) gebildet ist, um zu ermöglichen, dass ein zweites chirurgisches Instrument durch dieses hindurch eingeführt werden kann; und
ein Zylinderteil (17), das an dem Plattenelement (2) bereitgestellt ist, um mit dem Innern des ersten Durchgangslochs (3) und dem Innern des zweiten Durchgangslochs (12) zu kommunizieren, und das angepasst ist, um an einer Schnittwunde angebracht zu werden,
wobei das Plattenelement (2) ein Führungslochteil (6) aufweist, durch das der bewegbare Stutzen (11) eingeführt wird, wobei
das Führungslochteil (6) einen Langlochabschnitt (7) umfasst, der es ermöglicht, dass sich der bewegbare Stutzen (11) in einer vorbestimmten Richtung vor und zurück bewegt, wobei sich der Langlochabschnitt (7) von einem ersten Ende (7a) bis zu einem zweiten Ende (7b) des Langlochabschnitts (7) in einer Längsrichtung erstreckt, die zu einer mittleren Achse des Plattenelements (2) rechtwinklig ist,
**dadurch gekennzeichnet, dass** das Führungslochteil (6) ferner
einen Blockierungsabschnitt (8) umfasst, der konfiguriert ist, um den bewegbaren Stutzen (11) an einem Teil des Führungslochteils (6) zu blockieren, wobei der Blockierungsabschnitt (8) an einem Zwischenabschnitt des Langlochabschnitts (7) zwischen dem ersten und dem zweiten Ende des Langlochabschnitts (7) in der Längsrichtung gebildet ist, wobei
ein Gleitwiderstand in der Längsrichtung zwischen dem Langlochabschnitt (7) und dem bewegbaren Stutzen (11) an beiden Enden (7a, 7b) des Langlochabschnitts (7) im Vergleich zu einem Gleitwiderstand in der Längsrichtung zwischen dem Blockierungsabschnitt (8) und dem bewegbaren Stutzen (11) geringer ist.

2. Trokar nach Anspruch 1,
wobei das Führungslochteil (6) ferner ein Ventil (9) umfasst, das den Langlochabschnitt (7) luftdicht versiegelt, und
wobei ein Schlitz (10), durch den der bewegbare Stutzen (11) eingeführt wird und der sich in der Längsrichtung des Langlochabschnitts (7) erstreckt, in dem Ventil (9) gebildet ist.

## Revendications

1. Trocart (1) comprenant :
un élément plaque (2) qui a au moins un premier trou traversant (3) formé à l'intérieur de celui-ci pour permettre à un premier instrument chirurgical d'être introduit à travers celui-ci ;
au moins un orifice mobile (11) qui est prévu séparément de l'élément plaque (2), est mobile par rapport à l'élément plaque (2), et a un second trou traversant (12) formé à l'intérieur de celui-ci pour permettre à un second instrument chirurgical d'être introduit à travers celui-ci ; et
une partie cylindrique (17) qui est disposée au niveau de l'élément plaque (2) de façon à communiquer avec un intérieur du premier trou traversant (3) et un intérieur du second trou traversant (12), et qui est adaptée pour être attachée à une plaie incisée,
l'élément plaque (2) ayant une partie trou de guidage (6) à travers laquelle l'orifice mobile (11) est introduit,
la partie trou de guidage (6) comprenant une partie de trou allongée (7) qui permet à l'orifice mobile (11) d'avancer et de reculer dans une direction prédéterminée, la partie de trou allongée (7) s'étendant d'une première extrémité (7a) à une seconde extrémité (7b) de la partie de trou allongée (7) dans une direction longitudinale perpendiculaire à un axe central de l'élément plaque (2),
**caractérisé par le fait que** la partie trou de guidage (6) comprend en outre
une partie de verrouillage (8) qui est configurée pour verrouiller l'orifice mobile (11) à une partie de la partie trou de guidage (6), la partie de verrouillage (8) étant formée au niveau d'une partie intermédiaire de la partie de trou allongée (7) entre les première et seconde extrémités de la partie de trou allongée (7) dans la direction longitudinale,
une résistance au coulissement dans la direction longitudinale entre la partie de trou allongée (7) et l'orifice mobile (11) au niveau des deux extrémités (7a, 7b) de la partie de trou allongée (7) étant plus petite par comparaison à une résistance au coulissement dans la direction longitudinale entre la partie de verrouillage (8) et l'orifice mobile (11).

2. Trocart selon la revendication 1,
dans lequel la partie trou de guidage (6) comprend en outre une valve (9) qui scelle de manière étanche la partie de trou allongée (7), et
une fente (10), à travers laquelle l'orifice mobile (11) est introduit et qui s'étend dans la direction longitudinale de la partie de trou allongée (7), étant formée dans la valve (9).
